# EUROPEAN PATENT APPLICATION

(11) **EP 4 664 920 A1**
(43) Date of publication of application: **17.12.2025**
(21) Application number: 25182817.4
(22) Date of filing: 13.06.2025
(51) Int. Cl.: H04R 1/10, H04R 27/00, H04S 7/00

(54) **HEARING PROTECTION WITH DIRECTIONAL AUDIO**

(30) Priority: 14.06.2024 US 202463660025 P
(71) Applicant: Kotowick, Kyle, Ottawa, ON K1Y 2C5 (CA)
(72) Inventor: Kotowick, Kyle, Ottawa, ON K1Y 2C5 (CA)
(74) Representative: Dehns

(57) **Abstract**

Hearing protection (102) including passive noise cancellation or reduction material can include ultrawideband radios (110). The hearing protection may include a microphone (112a, 112b) for capturing the audio (106) when the user (104) is speaking. An ultrawideband radio (110) can be used to transmit the audio (106) to other users (104) with similar hearing protection (102). The ultrawideband radio (110) can also be used to determine a relative location of other users (104) and 3D spatial audio encoding can be applied to the associated audio signals in order to make it appear that the audio signals are coming from the relative location of the other respective users (104). The hearing protection (102) may be used in a wide range of environments, including for example for first responders. In such cases, the hearing protection (102) may include active noise cancelling functionality for reducing a loudness of siren noises.

## Description

### RELATED APPLICATIONS

The current application claims priority to US Provisional Application 63/660,025 filed June 14th, 2024 and titled "Hearing Protection With Directional Audio," the entire contents of which are incorporated herein by reference for all purposes.

### TECHNICAL FIELD

The current disclosure relates to hearing protection for use in loud environments and in particular to hearing protection with directional audio.

### BACKGROUND

It can be desirable, or required, for individuals in loud environments to wear some form of hearing protection. These may be over-the-ear devices or in-ear devices that provide passive noise protection that reduces the sound heard by the wearer. Such passive hear protection will block, or reduce the sound of, all sounds, including potentially wanted sounds such as speaking.

Active noise cancelling devices reduce the loudness of sounds heard by a wearer by essentially playing a 180° out of phase sound to the sound being cancelled. Active noise cancellation works well for reducing relative constant noise, such as engine or motor noises but is not as effective at reducing varying noises such as speaking, hammering, sirens, etc.

Alternative, additional and/or improved hearing protection devices are desirable.

### SUMMARY

In accordance with the present disclosure there is provided a device for hearing protection comprising: a speaker associated with an ear of a user; at least one microphone; a first locating radio; a processor for executing instructions; and a memory storing instructions which when executed configure the device to: receive transmissions from a locating radio transmitter of a second device for hearing protection, at least a portion of the received transmissions encoding an audio signal captured at the second device for hearing protection; determining a relative location of the second device for hearing protection from the received transmissions; applying 3D spatial audio encoding to the audio signal; and playing the 3D spatial audio encoded audio signal through the speaker.

In a further embodiment of the device, the locating radio comprises an ultra wide band (UWB) radio.

In a further embodiment of the device, determining the relative location of the second device for hearing protection further comprises receiving transmissions from a UWB anchor at a fixed location in the environment.

In a further embodiment of the device, the device further comprises a second UWB radio arranged away from the first UWB radio, wherein determining the relative location of the second device for hearing protection further comprises determining the relative location using the transmissions received from the first UWB radio and transmissions received from the second UWB radio.

In a further embodiment of the device, the instructions when executed configure the device to provide one or more of: active noise cancellation (ANC); and noise reduction and removal.

In a further embodiment of the device, the ANC comprises functionality for reducing noises from sirens.

In a further embodiment of the device, the functionality for reducing noises from sirens comprises: determining if siren noise is present in the audio signal; when it is determined siren noise is present in the audio signal, reducing a loudness of the siren noise at one or more predefined frequencies.

In a further embodiment of the device, reducing the loudness of the siren noise comprises removing or reducing the siren noise in the audio signal played back on the speaker.

In a further embodiment of the device, reducing the loudness of the siren noise comprises playing a cancellation signal at one or more of the predefined frequencies 180° out of phase from the siren noise.

In a further embodiment of the device, determining if siren noise is present comprises processing the audio signal to determine if the siren noise is present.

In a further embodiment of the device, determining if siren noise is present comprises receiving an indication from a vehicle that a siren is turned on.

In a further embodiment of the device, the instructions when executed configure the device to further: record audio from the microphone; and transmit the audio using the locating radio.

In a further embodiment of the device, the instructions when executed configure the device to further: identify a locating radio transmitter from the received transmissions.

In a further embodiment of the device, the instructions when executed configure the device to further: determine if the identified locating radio transmitter should be added to an active group of transmitters, the active group providing locating radio transmitters whose transmitted audio should be played to the user.

In a further embodiment of the device, the instructions when executed configure the device to further: determine user preferences associated with the identified locating radio transmitter; and apply the user preferences to the audio signal.

In a further embodiment of the device, the device further comprises one or more of: a Bluetooth radio; a LoRa radio; a WiFi radio; and a cellular radio.

In a further embodiment of the device, the device further comprises: noise dampening material to reduce sound of an environment.

In accordance with the present disclosure there is further provided a device for hearing protection comprising: noise dampening material to reduce sound of an environment; a speaker associated with an ears of a user; at least one microphone; a processor for executing instructions; and a memory storing instructions which when executed configure the device to: receive an audio signal; determining if siren noise is present in the audio signal; removing or reducing the siren noise from the audio signal; and playing the audio signal through the speaker.

In a further embodiment of the device, removing or reducing the siren noise comprises playing a cancellation signal at one or more of the predefined frequencies 180° out of phase from the siren noise.

In a further embodiment of the device, determining if siren noise is present comprises processing the audio signal to determine if the siren noise is present based on known siren frequencies.

In a further embodiment of the device, determining if the siren noise is present comprises receiving an indication from a vehicle that a siren is turned on.

In a further embodiment of the device, the received audio signal is received from the at least one microphone.

In a further embodiment of the device, the device further comprises a radio receiver, wherein the received audio signal is received from the radio receiver.

In a further embodiment of the device, the radio receiver comprises a locating radio.

In a further embodiment of the device, the locating radio comprises an ultra wide band (UWB) radio.

In a further embodiment of the device, the instructions stored in the memory, when executed, further configure the device to: receive transmissions from a locating radio transmitter of a second device for hearing protection, at least a portion of the received transmissions encoding an audio signal captured at the second device for hearing protection; determining a relative location of the second device for hearing protection from the received transmissions; applying 3D spatial audio encoding to the audio signal; and playing the 3D spatial audio encoded audio signal through the speaker.

In a further embodiment of the device, determining the relative location of the second device for hearing protection further comprises receiving transmissions from a UWB anchor at a fixed location in the environment.

In a further embodiment of the device, the device further comprises a second UWB radio arranged away from the first UWB radio, wherein determining the relative location of the second device for hearing protection further comprises determining the relative location using the transmissions received from the first UWB radio and transmissions received from the second UWB radio.

In a further embodiment of the device, the instructions when executed further configure the device to provide one or more of: active noise cancellation (ANC); and noise reduction and removal.

In a further embodiment of the device, the instructions when executed configure the device to further: record audio from the microphone; and transmit the audio using the locating radio.

In a further embodiment of the device, the instructions when executed configure the device to further: identify a locating radio transmitter from the received transmissions.

In a further embodiment of the device, the instructions when executed configure the device to further: determine if the identified locating radio transmitter should be added to an active group of transmitters, the active group providing locating radio transmitters whose transmitted audio should be played to the user.

In a further embodiment of the device, the instructions when executed configure the device to further: determine user preferences associated with the identified locating radio transmitter; and apply the user preferences to the audio signal.

In a further embodiment of the device, the device further comprises one or more of: a Bluetooth radio; a LoRa radio; a WiFi radio; and a cellular radio.

In a further embodiment of the device, the device further comprises: noise dampening material to reduce sound of an environment.

### BRIEF DESCRIPTION OF THE DRAWINGS

Further features and advantages of the present disclosure will become apparent from the following detailed description, taken in combination with the appended drawings, in which:
FIG. 1 depicts a hearing protection system;
FIG. 2 depicts a further hearing protection system;
FIG. 3 depicts a method for providing a hearing protection device with directional sound;
FIG. 4 depicts a method of providing hearing protection according to user preferences;
FIG. 5 depicts groups of users utilizing hearing protection devices;
FIG. 6 depicts a method for hearing protection in groups of users;
FIG. 7 depicts a hearing protection system for first responders;
FIG. 8 depicts a method of providing active siren cancellation for hearing protection; and
FIG. 9 depicts a further method of providing active siren cancellation for hearing protection.

### DESCRIPTION

Hearing protection is important for individuals that may be exposed to loud noises for sustained periods of time. While hearing protection exists that can reduce the decibel level of loud environments experienced by individuals, such hearing protection may also reduce the loudness of desirable sounds such as speech from others in the environment. For example, it may be necessary or desirable for workers in a loud environment to communicate with each other. Passive noise reduction will reduce the volume of the speakers as well as the noise of the environment. The amount of noise reduction provided can be varied depending upon the desired application. Active noise cancellation techniques may be helpful in these situations, however they reduce the loudness of generally constant noise such as motors, engines, etc. Further, active noise cancellation techniques will not work without power and as such if batteries fail, the hearing protection will no longer be available. Hearing protection using passive noise reduction may include speakers that allow sound to be played to the user. While these hearing protection devices can be used to playback sound, such as music or possibly the speaking of another user, they can be disorienting as the sound of the speaker does not appear to originate from the speaker. As described further below, hearing protection devices can be provided with an ultrawideband (UWB) radio that can be used to determine a relative location of other UWB transmitters. The hearing protection devices may include one or more wireless radios for transmitting and/or receiving audio or possibly other data. The UWB radio may also be used to transmit and/or receive audio or possibly other data. The determined locations of other hearing protection devices which may be transmitting audio may then be used to encode the audio signal using 3D spatial audio encoding techniques so that the received audio will appear to originate from the associated transmitting location or locations.

While hearing protection in loud environments generally reduces the sound level of the environment, other applications may reduce the sound level of particular noises. As described further herein, hearing protection may be particularly useful to first responders, or other individuals exposed to loud sirens. The hearing protection for such applications may include functionality for reducing the sound of the sirens. Reducing siren noises can be difficult for active noise cancellation techniques as it can be computationally expensive to identify the siren frequencies and determine a cancellation signal, which when played back would combine with the siren noise to reduce the sound heard by the user. As described further below, the current hearing protection can use a set of predefined frequencies associated with sirens to quickly determine the cancellation signal for cancelling or reducing the siren noise. For passive noise reduction devices that include audio playback, while the siren noises may be significantly reduced by the passive noise reduction, it is possible that the audio will capture siren noises. Similar audio processing techniques to identify the siren and remove it from the audio signal prior to being played back can eliminate, or reduce, the sound of the siren.

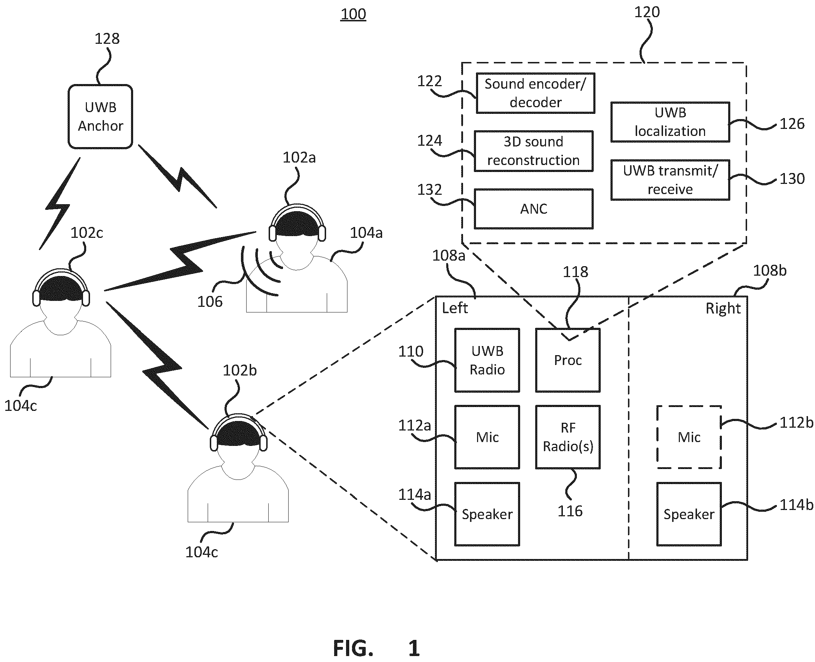

FIG. 1 depicts a hearing protection system. The system 100 comprises a plurality of hearing protection devices 102a, 102b, 102c (referred to collectively as hearing protection devices 102) that are each associated with respective users 104a, 104b, 104c (referred to collectively as users 104) in an environment. One or more of the users 104 may be speaking with or to one or more of the other users 104, depicted graphically by sounds 106a. The hearing protection devices 102 reduce loud sounds of the environment while allowing users 104 to communicate with each other. Further, the hearing protection devices can use 3D spatial audio techniques in order to localize audio associated with other users.

Each of the hearing protection devices are depicted as over-the-ear devices with two ear cups connected by a headband. The hearing protection devices may also be provided by in-ear devices that comprise respective ear buds or similar structures. The individual ear buds may or may not be connected together. Regardless of the exact form factor of the hearing protection devices 102, they provide some level noise reduction, which may be active or passive. Passive noise reduction may be provided by material that reduces the sound level of all sound frequencies or a range or ranges of frequencies, while active noise cancellation techniques can reduce sound levels, typically of generally constant noise, by detecting a sound or noise to be cancelled and playing a corresponding signal that is approximately 180° out of phase in order to cancel or reduce the sound or noise as heard by the user. In addition to reducing the sound levels for users, the hearing protection devices may allow individual users to communicate with each other. The devices 102 may capture speech of the users and transmit the signals to the other hearing protection devices 102 for playback. The speech may be played back using 3D spatial audio techniques that allow the speech to seem to the listener to originate from a particular spatial location, which may be the location of the user associated with the speech being played back.

The hearing protection devices 102 may comprise a left ear device 108a and a right ear device 108b that house the various components of the hearing devices. It will be appreciated that although depicted as being arranged in ear portions, it is possible to incorporate certain portions of the electronics in other portions such as a headband. Further, while certain components are depicted as being in one of the ear portions, it is possible to include components in either or both of the ear components. The hearing devices are depicted as comprising an ultrawideband (UWB) radio 110, a microphone 112a, and possibly a second microphone 112b in the other ear portion or at other locations in the devices 102 such as near a user's mouth. Speakers 114a, 114b are arranged to playback sounds to the user's left and right ears respectively. Although depicted as an individual speaker, it is possible that multiple speakers may be associated with each ear. The hearing devices 102 may also include one or more additional RF radios 116, which may include for example a Bluetooth radio, a WiFi radio, and/or a cellular radio. Further RF radios may be incorporated as well. The RF radios may allow additional functionality such as remote communications with others, connecting to a user's mobile phone, connecting to other systems such as vehicles or other devices in the environment. Such connections may be useful in providing additional information to users, including for example possibly playing back to the user announcements or other information that may have been blocked or reduced in volume by the hearing protection devices.

The hearing protection devices 102 further include a processor 118 that executes instructions from a memory. The memory may be part of the processor and/or may include separate memory (not shown) that is separate from the processor. When the instructions are executed by the processor, the hearing devices are configured to provide various functionality 120. The functionality may include, for example, sound encoder / decoder functionality 122 that can encode audio signals for subsequent transmission as well as decoding received audio signals. 3D sound reconstruction functionality 124 may use 3D spatial audio techniques to encode the audio signals so that when played back to the user, spatial audio appears to originate from a particular spatial location. In order to provide the spatial audio, the 3D sound reconstruction functionality 124 requires an indication of the spatial location that the audio signal being encoded is supposed to originate from. The relative location of the user, or rather the hearing protection device of a user, can be determined using a locating radio that can determine locations based on received transmissions from other locating radios. The locating radio is described further below as a UWB radio; however other RF radios may be used. The locating radios may use multiple antennas and time-difference-of-arrival or phase-difference-of-arrival techniques in order to determine the relative locations. While the locating radio is used to determine relative locations it is possible for the locating radios to also transmit and/or receive audio, video and/or data signals.

The relative location of the user, or rather the hearing protection device of a user, can be determined using the UWB radio transmissions that are received. UWB localization functionality 126 may be used to determine the relative location of other UWB transmitters based on the received UWB signals. With a single UWB radio, or more particularly an antenna receiving a UWB transmission at a single location, the localization may only be able to determine a distance to a transmitter and not a direction. In order to determine the location, namely the distance and direction from a UWB radio with a single antenna in the hearing protection devices, one or more UWB anchors 128 may be arranged in the environment. The UWB anchors 128 may be arranged at known locations and can be used when determining relative locations of UWB transmitters of devices 102.

The hearing protection devices 102 include UWB transmit and receive functionality 130 that use the UWB radio to transmit and receive UWB signals. The UWB signals may include, for example, audio captured by the microphones of the devices, which may capture for example a user's speech. The UWB radios may transmit additional information to other devices other than the audio. The UWB radios are capable of transmitting and receiving audio signals simultaneously in order to provide simultaneous two-way communication between users of the hearing protection devices.

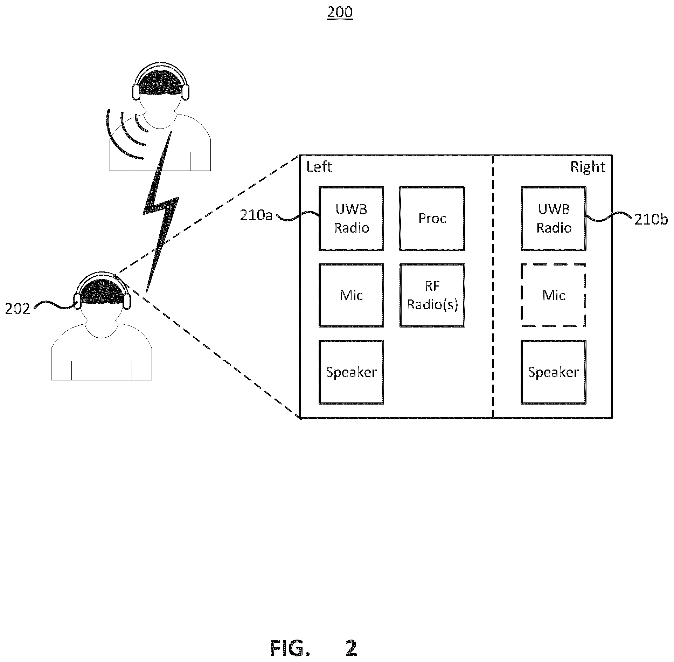

FIG. 2 depicts a further hearing protection system. The system 200 is similar to that of the system 100 described above, and similar components and functionality are not described further. In the system 100, the hearing protection devices include a single UWB radio, or more particularly a single antenna, and rely on one or more UWB anchors to determine relative locations of UWB transceivers. In contrast to the system 100, the system 200 does not require the use of UWB anchors in order to determine relative locations of transmitters. Instead the hearing protection devices 202, includes at least two separate UWB radios 210a, 210b, which allow the relative locations of other hearing protection devices to be determined. It is noted that FIG. 2 depicts two separate UWB radios, however the device may comprise a single UWB radio with multiple antennas spaced apart from each other by a known distance. Further, the device may include multiple UWB radios each with multiple antennas such as 3 UWB radios with 2 antennas each. With the two UWB radio antennas, the hearing protection device 202 can use phase difference of arrival, time difference of arrival, or a combination of these techniques to determine the location, such as the direction and distance, of a transmitter based on the phase or time difference between the signal received at the different UWB radios 210a, 210b. While both UWB radios 210a, 210b may be used for receiving transmitted signals, one may be used for transmitting a particular UWB signal. When multiple UWB radios are present, more than one of the multiple UWB radios may transmit simultaneously. For example, one transmission may be used for ranging or direction finding while other UWB radios may be used for transmitting audio, or data.

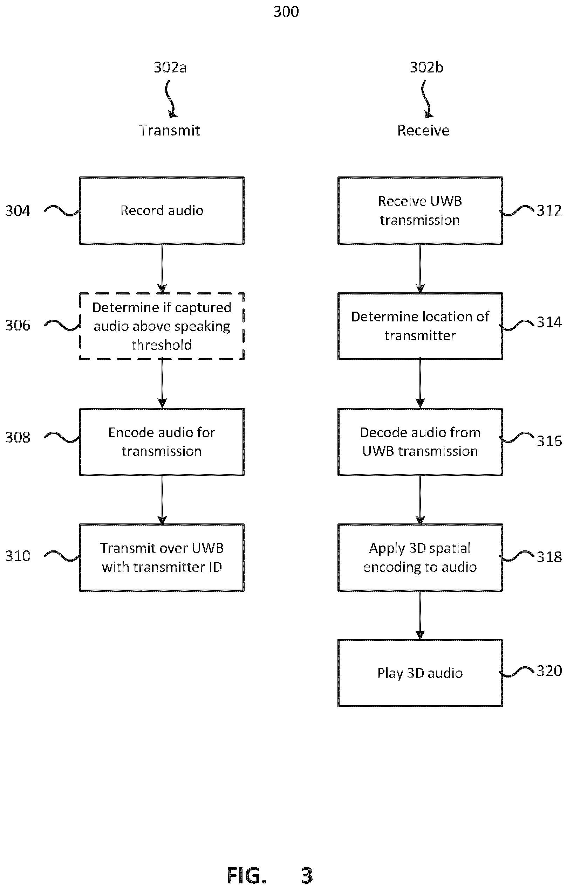

FIG. 3 depicts a method for providing a hearing protection device with directional sound. The method 300 includes a transmit portion 302a, and a receive portion 302b. The transmit and receive portions may be performed substantially simultaneously in order to allow two-way communication between users. The transmit portion 302a begins with capturing or recording audio from the microphone, or microphones of the hearing protection device (302). The audio signal may be optionally processed in order to determine if there is audio above a speaking threshold (306). This may be done in order to transmit audio only when the user is speaking. The captured audio is encoded (308) and then transmitted over the UWB radio with an identification of the transmitter (310) so that the particular transmitter associated with the audio can be determined by the receiver. It is possible for the audio to be transmitted over one or more different radios besides the UWB radio.

At the receive end 302b, the UWB transmission is received (312) and used to determine the location of the transmitter (314). The location determination may use additional signals received from other transmitters such as UWB anchors, and/or from other UWB receivers within the same hearing protection device. The audio from the received UWB transmission is decoded (316) and then the determined location of the associated UWB transmitter used to apply 3D spatial encoding to the audio (318) in order to make it seem to originate from the determined location. The spatial audio is then played back to the user 320. The hearing devices may perform other processing on the audio, including for example combining with other received audio, increasing or decreasing a volume of the audio, filtering the audio etc.

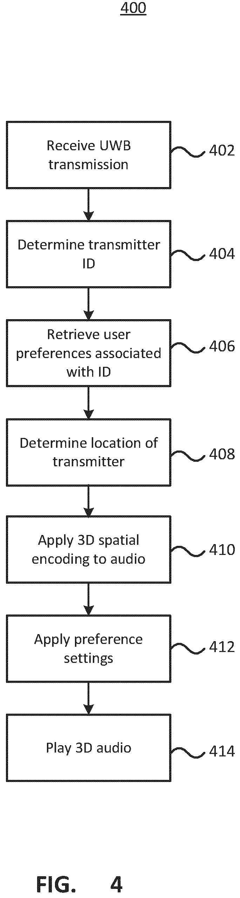

FIG. 4 depicts a method of providing hearing protection according to user preferences. The method 400 receives a UWB transmission (402) and determines a transmitter ID associated with the transmission (404). The ID is used to retrieve user preferences stored in association with the ID (406). The preferences may include for example indications of user preferences such as a preferred volume of audio from the transmitter, a priority of the audio from the transmitter which may be used to preferentially play audio from the transmitter instead of or louder than another transmitter. Other preferences may be specified that provide an indication as to how audio from the transmitter should be processed and played back to the user. In addition to determining an ID with the transmission, the UWB transmission is used to determine a relative location of the transmitter (408). The determined location is used to apply 3D spatial audio encoding to the audio signal (410) from the received UWB transmission. The retrieved user preferences can be applied to the spatial audio (412), or possibly applied prior to the spatial encoding, and the audio can be played back to the user (414). The preferences may be specified in various ways, including for example using an application on a user's mobile device that can communicate the user preference settings to the hearing protection device of the user. Other preferences, including for example specifying one or more groups a user may participate in, can be specified.

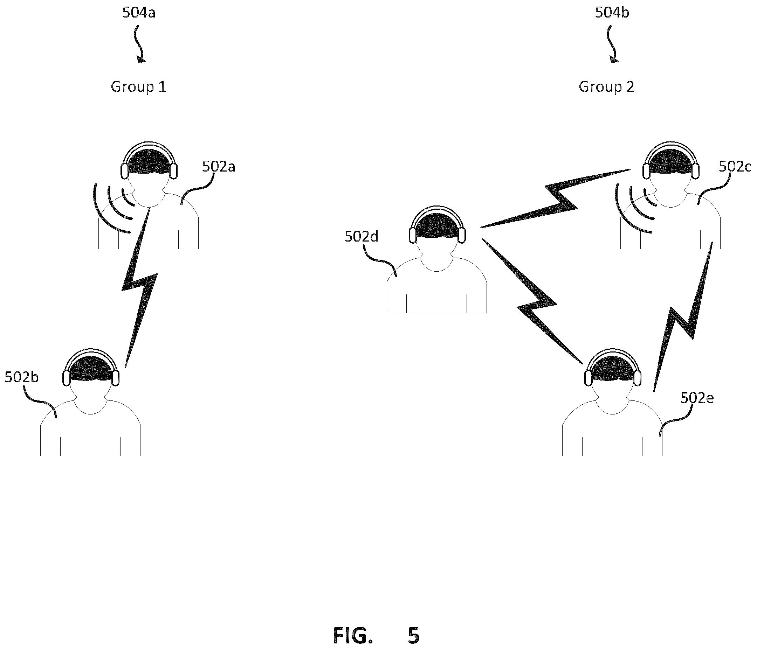

FIG. 5 depicts groups of users utilizing hearing protection devices. The above has described the ability of different users to communicate with each other while using hearing protection devices that reduce the sounds in the environment. In the above, it was assumed that all users may transmit/receive audio to/from all other devices in the vicinity. This may be desirable in certain environments. However, in other environments it may be desirable to allow users to include, or exclude, others from the audio they hear. As depicted in FIG. 5 a number of individuals 502a..e may be in the same environment. Each of the users 502a..e may have their own hearing protection devices. As depicted, the users may form 2 groups 504a, 504b, although any number of groups may be used, of users. Each of the users of a group may hear the users within the same group, while users in the other group are muted. That is, the speech of a user in one group may be played back, with appropriate 3D spatial encoding, to users within the same group, while users in other groups may not playback the speech and so not hear the users of different groups. While FIG. 5 depicts users as being in a single group, it is possible for users to be in one or more groups simultaneously.

Although not depicted in FIG. 5, the hearing protection devices may apply encryption techniques to the audio transmitted between users so that only users within the same group can decode and listen to the audio. It will be appreciated that encryption techniques may be applied within groups, or may be applied for all devices, or subsets of devices whether arranged in groups or not.

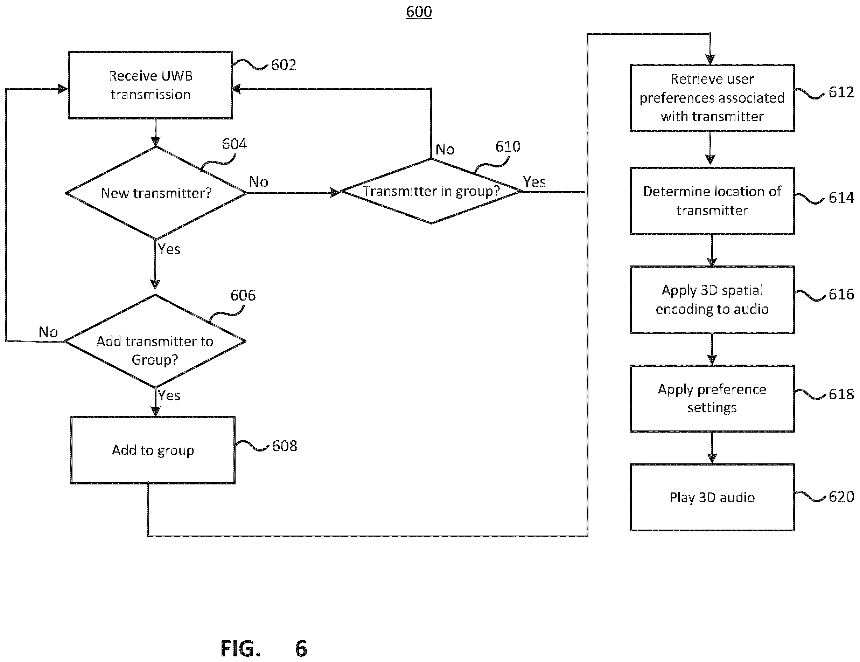

FIG. 6 depicts a method for hearing protection in groups of users. The method 600 receives a UWB transmission (602) and determines if it is from a new transmitter (604). If it is a newly identified transmitter (Yes at 604) it is determined if the transmitter should be added to the group of the current transmitter (606). Although depicted as a single group, it is possible for the current hearing protection device to be a member of multiple groups and the newly identified transmitter would be checked to determine if it should be added to each of the groups of the user or more particularly the hearing protection device associated with the user. If the new transmitter is not to be added to the group (No at 604) it can continue waiting to receive further UWB transmission. If the transmitter is to be added to the group (Yes at 606), it is added to the group (608). If the UWB transmission is not from a new transmitter, that is it is from a previously identified transmitter, it is determined if the transmitter is in the group (610). If it is not in the group, or groups, of the current hearing protection device (no at 610), the method may continue to wait for further UWB transmissions (602). If the transmitter is in the group (Yes at 610), or it has just been added to the group (608), preferences associated with the transmitter, or a user associated with the transmitter, can be retrieved (612). A location of the UWB transmitter is determined (614) and used to apply 3D spatial audio encoding to the audio from the received UWB transmission (616). The retrieved preferences can be applied (618) to the spatial audio, or possibly before the spatial audio encoding, and the resulting audio played back to the user (620).

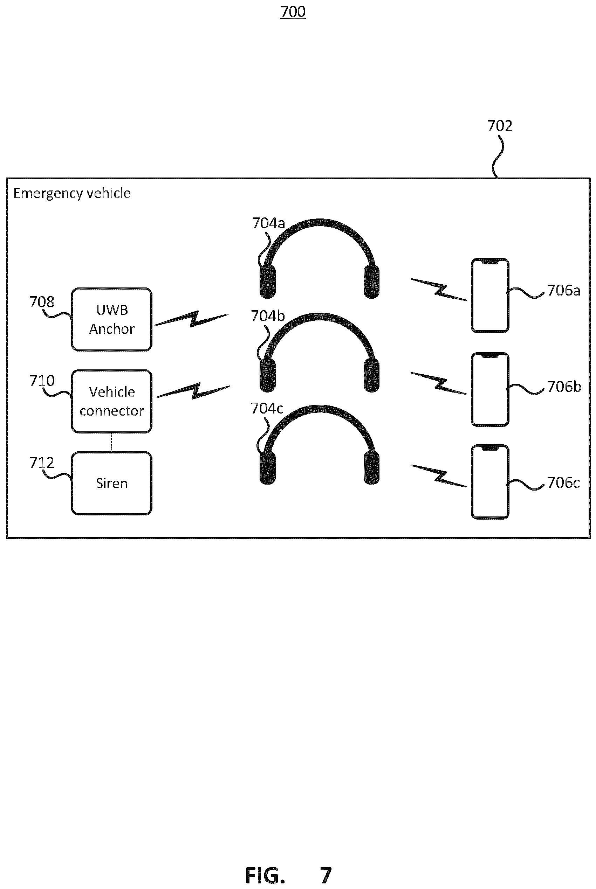

FIG. 7 depicts a hearing protection system for first responders. The above has described various embodiments for hearing protection devices. While the devices can be used in a wide range of applications, one particular application is for example for first responders, or other individuals that are exposed to siren noises. Since first responders often travel in emergency vehicles with the sirens on, which can be loud and cause hearing damage to the first responders, the hearing protection devices can be particularly useful to them. While the hearing protection devices may provide passive noise reduction protection to reduce the sound of sirens, they devices may additionally, or alternatively, include active noise cancellation to reduce the sound of the sirens, while allowing the first responders to communicate with each other. Using active noise cancellation techniques to reduce the noise of sirens can be difficult as the siren is not a constant noise, and as such the cancelling signal to play to reduce or cancel the siren can be computationally difficult to determine. In order to achieve the siren noise cancellation in a device with limited processing power, such as hearing protection devices, the cancelling signal can be selected from one or more known, predefined frequencies that first responder sirens operate at such as 700Hz and 950Hz. The particular frequencies used may depend on the siren type and the location. Since the siren frequencies are predefined, they can more easily be played back 180° out of phase from the siren in order to reduce or eliminate the sound of the siren heard by the first responders.

As depicted, the first responder system may include an emergency vehicle 702 which may transport one or more first responders or individuals. One or more hearing protection devices 702a..702c may be used by the individuals in the vehicle. It is noted that while described as being used within the vehicle, the hearing protection devices may also be used outside the vehicle in the vicinity of the vehicle. The hearing protection devices may be associated with the vehicle and/or may be associated with the users. Each of the hearing protection devices may be associated with one or more mobile phones or devices 704a..704c. For example, each user may associate their hearing protection devices with their respective mobile phone. The emergency vehicle may include one or more UWB anchors 706 to support the localization of the individual headsets. The localization may be used in order to adjust audio from other headsets in order to make it appear that the audio is coming from the speaker's location. Such localization of the audio associated with respective hearing protection devices may be helpful to first responders situational awareness and make communication more effective.

The system may further include a vehicle connector 708 that allows the hearing protection devices 702a..702c to be connected to the vehicle and vehicle systems by a wired or wireless connection. This may allow, for example, notification of information received at the vehicle that can be played back to the users. The information may further include information about the state of vehicle systems which may be used to adjust operation of the hearing protection devices. As an example, the vehicle connector may be coupled to the vehicle's siren in order to provide an indication to the hearing protection devices when the vehicle's siren is on and/or off. Such notifications may further simplify the processing performed by the hearing protection devices as it is not necessary to process the audio captured by the hearing protection devices in order to determine if a siren is on.

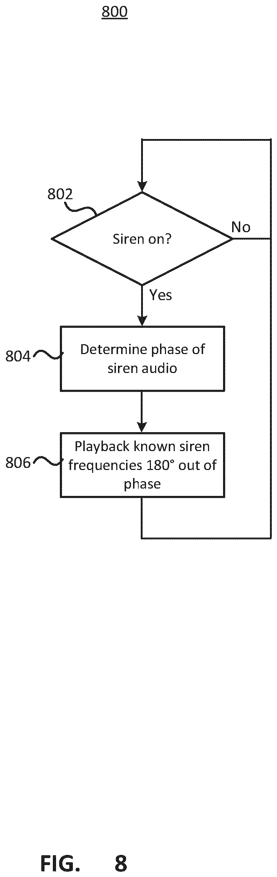

FIG. 8 depicts a method of providing active siren cancellation for hearing protection. The method 800 uses noise cancellation techniques in order to reduce or eliminate the siren sound heard by users of the hearing protection devices. The method 800 determines if the siren is on (802). The determination may be done in various ways. For example, the hearing protection device may receive a signal indicative that the siren is on or off. Such a signal may be communicated to the hearing protection device from the vehicle or siren system. Additionally, or alternatively, determining whether the siren is on or off may use audio processing techniques. For example, the siren detection may be performed by applying a Fourier transform to the audio from the microphone of the hearing protection device, and then determining if the amplitude of signals at known predefined frequencies associated with sirens is above a threshold. When the frequency, or frequencies, are above the amplitude threshold, it may be determined that the siren is on. Regardless of how the determination that the siren is on is made, once it is determined that the siren is on (Yes at 802), the phase of the siren audio is determined (804) and a cancellation signal comprising the siren frequencies is played back to the user 180° out of phase from the siren audio (806). The cancellation signal and the siren audio combine in a manner to at least partially cancel each other out and so reduce the overall loudness of the siren that the individuals using the hearing protection devices are exposed to. The cancellation signal may be continued to be played back to the user until the siren is no longer on, or some other stopping signal is received. Further, it is possible to provide additional tuning controls to the user, for example through their mobile device, or one or more controls on the hearing protection devices. The tuning controls may allow the user to adjust the phase of the cancellation signal and/or the frequencies, in order to provide fine-grained control to the user.

The above has described siren cancellation for active noise cancellation devices by identifying siren frequencies at known frequencies and then playing back the known siren signals 180° out of phase in order to destructively interfere with the siren sound. While such techniques may work with active noise cancellation techniques, it is possible for the siren sound to be picked up microphones capturing audio signals that may be played back to the user. For example, in the case of two first responders in an emergency vehicle with the siren on, the microphone used to pick up the user's speech and play it back to the other users may pick up the siren sound. It is possible to apply similar audio processing techniques in order to identify the siren sound in the audio and remove it, or at least reduce the loudness of the siren, in the audio being played back to users.

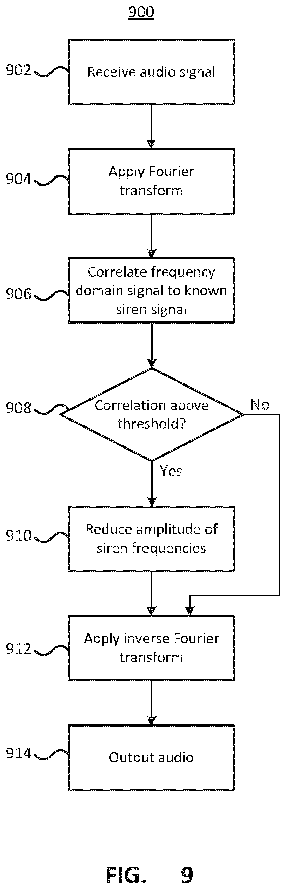

FIG. 9 depicts a method for reducing siren noise from an audio signal. The method 900 may be used to reduce, or remove, siren noises from an audio signal that is played back on hearing protection devices. The hearing protection devices may be active or passive hearing protection devices that allow playback of spatial audio to the wearer, such as speech captured from other hearing protection devices. The method 900 receives an audio signal (902). The audio signal may be captured from other hearing protection devices that capture and transmit speech to other hearing protection devices. The captured audio signal may capture siren audio which would otherwise be played back to the wearer, but may be removed from the audio signal as described further below. The audio signal is transformed into the frequency domain using a Fourier transform (904), which may possibly use a Fast Fourier Transform (FFT). The frequency domain audio signal is correlated with frequency domain signals of one or more known siren signals (906). The known siren signals may be based on the siren of the vehicle the user is in, sirens signals known to be used in the region of the user, or other siren signals that may be used. If the siren picked up in the audio signal is from a vehicle the user is in, there will be no Doppler shift in the siren as there is no relevant movement between the user and the siren. If however there is movement between the user and the siren, it is possible that the correlation may also account for the possible Doppler shifted signals. Based on the correlation, it is determined if a siren signal is present. For example, the correlation at siren frequencies, or frequency, can be evaluated to determine if it exceeds a threshold (908). If the correlation is above a threshold (Yes at 908) and so there is a siren in the audio, the amplitude of the siren signals is reduced or removed (910). With the siren removed, or its loudness reduced, an inverse Fourier transform can be applied (912) in order to return the frequency domain signal to the time domain and the audio signal output (914). The audio signal may be a spatially encoded audio signal, or the output signal with the siren noise removed or reduced, can be spatially encoded.

The siren noise reduction can be used with passive noise cancellation devices that include speech capture and playback capabilities. Furthermore, the siren noise reduction from the audio signal may be used with active noise cancellation devices. The siren noise reduction from the audio signal may be used in conjunction with the location determination and 3D spatial encoding of audio signals, or may be used with noise protection devices that do not include spatial encoding.

It will be appreciated by one of ordinary skill in the art that the system and components shown in FIGs. 1 - 9 can include components not shown in the drawings. For simplicity and clarity of the illustration, elements in the figures are not necessarily to scale, are only schematic and are non-limiting of the elements structures. It will be apparent to persons skilled in the art that a number of variations and modifications can be made without departing from the scope of the invention as defined in the claims.

Although certain components and steps have been described, it is contemplated that individually described components, as well as steps, can be combined together into fewer components or steps or the steps can be performed sequentially, non-sequentially or concurrently. Further, although described above as occurring in a particular order, one of ordinary skill in the art having regard to the current teachings will appreciate that the particular order of certain steps relative to other steps can be changed. Similarly, individual components or steps can be provided by a plurality of components or steps. One of ordinary skill in the art having regard to the current teachings will appreciate that the components and processes described herein can be provided by various combinations of software, firmware and/or hardware, other than the specific implementations described herein as illustrative examples.

The techniques of various embodiments can be implemented using software, hardware and/or a combination of software and hardware. Various embodiments are directed to apparatus, e.g. a node which can be used in a communications system or data storage system. Various embodiments are also directed to non-transitory machine, e.g., computer, readable medium, e.g., ROM, RAM, CDs, hard discs, etc., which include machine readable instructions for controlling a machine, e.g., processor to implement one, more or all of the steps of the described method or methods.

Some embodiments are directed to a computer program product comprising a computer-readable medium comprising code for causing a computer, or multiple computers, to implement various functions, steps, acts and/or operations, e.g. one or more or all of the steps described above. Depending on the embodiment, the computer program product can, and sometimes does, include different code for each step to be performed. Thus, the computer program product may, and sometimes does, include code for each individual step of a method, e.g., a method of operating a communications device, e.g., a wireless terminal or node. The code can be in the form of machine, e.g., computer, executable instructions stored on a computer-readable medium such as a RAM (Random Access Memory), ROM (Read Only Memory) or other type of storage device. In addition to being directed to a computer program product, some embodiments are directed to a processor configured to implement one or more of the various functions, steps, acts and/or operations of one or more methods described above. Accordingly, some embodiments are directed to a processor, e.g., CPU, configured to implement some or all of the steps of the method(s) described herein. The processor can be for use in, e.g., a communications device or other device described in the present application.

Numerous additional variations on the methods and apparatus of the various embodiments described above will be apparent to those skilled in the art in view of the above description. Such variations are to be considered within the scope.

## Claims

1. A device for hearing protection comprising:
a speaker associated with an ear of a user;
at least one microphone;
a first locating radio;
a processor for executing instructions; and
a memory storing instructions which when executed configure the device to:
receive transmissions from a locating radio transmitter of a second device for hearing protection, at least a portion of the received transmissions encoding an audio signal captured at the second device for hearing protection;
determining a relative location of the second device for hearing protection from the received transmissions;
applying 3D spatial audio encoding to the audio signal; and
playing the 3D spatial audio encoded audio signal through the speaker.

2. A device for hearing protection comprising:
noise dampening material to reduce sound of an environment;
a speaker associated with an ears of a user;
at least one microphone;
a processor for executing instructions; and
a memory storing instructions which when executed configure the device to:
receive an audio signal;
determining if siren noise is present in the audio signal;
removing or reducing the siren noise from the audio signal; and
playing the audio signal through the speaker.

3. The device of claim 2, further comprising a locating radio.

4. The device of claim 3, wherein the instructions stored in the memory, when executed, further configure the device to:
receive transmissions from a locating radio transmitter of a second device for hearing protection, at least a portion of the received transmissions encoding an audio signal captured at the second device for hearing protection;
determining a relative location of the second device for hearing protection from the received transmissions;
applying 3D spatial audio encoding to the audio signal; and
playing the 3D spatial audio encoded audio signal through the speaker.

5. The device of claim 1 or 4, wherein the locating radio comprises an ultra wide band (UWB) radio.

6. The device of claim 5, wherein determining the relative location of the second device for hearing protection further comprises receiving transmissions from a UWB anchor at a fixed location in the environment.

7. The device of claim 6, further comprising a second UWB radio arranged away from the first UWB radio, wherein determining the relative location of the second device for hearing protection further comprises determining the relative location using the transmissions received from the first UWB radio and transmissions received from the second UWB radio.

8. The device of any one of claims 1 to 7, wherein the instructions when executed further configure the device to provide one or more of:
active noise cancellation (ANC); and
noise reduction and removal.

9. The device of claim 8 when dependent upon claim 1, wherein the ANC comprises functionality for reducing noises from sirens.

10. The device of claim 9, wherein the functionality for reducing noises from sirens comprises:
determining if siren noise is present in the audio signal;
when it is determined siren noise is present in the audio signal, reducing a loudness of the siren noise at one or more predefined frequencies.

11. The device of claim 10, wherein reducing the loudness of the siren noise comprises removing or reducing the siren noise in the audio signal played back on the speaker.

12. The device of claim 11 or 2, wherein reducing the loudness of the siren noise comprises playing a cancellation signal at one or more of the predefined frequencies 180° out of phase from the siren noise.

13. The device of claim 12, wherein determining if siren noise is present comprises at least one of:
processing the audio signal to determine if the siren noise is present; and
receiving an indication from a vehicle that a siren is turned on.

14. The device of any one of claims 1 to 13, wherein the instructions when executed configure the device to further:
record audio from the microphone; and
transmit the audio using the locating radio.

15. The device of any one of claims 1 to 14, further comprising one or more of:
a Bluetooth radio;
a LoRa radio;
a WiFi radio; and
a cellular radio.
